# FASCICULE DE BREVET EUROPEEN

(11) **EP 0 941 724 B1**
(45) Date de publication et mention de la délivrance du brevet: **13.08.2003**
(21) Numéro de dépôt: 99400534.6
(22) Date de dépôt: 05.03.1999
(51) Int. Cl.: A61F 5/448

(54) **Appareillage pour stomie**
Stomavorrichtung
Stoma apparatus

(30) Priorité: 12.03.1998 FR 9803061
(43) Date de publication de la demande: 15.09.1999
(73) Titulaire: B. BRAUN BIOTROL, 92107 Boulogne Billancourt (FR)
(72) Inventeur: Holtermann, Henri, 64500 Saint Jean de Luz (FR)
(74) Mandataire: Vialle-Presles, Marie José

(56) Documents cités:
- EP-A- 0 433 102
- EP-A- 0 799 608
- DE-B- 1 266 929
- FR-A- 1 156 435

## Description

La présente Invention se rapporte à un raccord propre à entrer dans la constitution d'un appareillage pour stomie ainsi qu'à un appareillage pour stomie comprenant un tel raccord.

De nombreux appareillages ont déjà été proposés pour assurer le recueil des matières corporelles (fèces, urine, ...) chez des personnes ayant subi une intervention chirurgicale du tractus gastro-intestinal de type iléostomie, colostomie, ou de l'appareil urinaire de type urostomie, ou une intervention chirurgicale nécessitant le drainage de plaies ou de cavités infra-abdominales du type drainage péritonéal ou sous-péritonéal. Parmi ces appareillages, on distingue schématiquement :
- des appareillages dits "une-pièce" car ils sont constitués par une poche de recueil qui comprend un protecteur cutané muni d'un adhésif sensible à la pression et qui est destinée à être fixée sur la peau de l'utilisateur par l'intermédiaire de ce seul protecteur cutané, et
- des appareillages dits "deux-pièces" qui comprennent une plaque frontale ou "porte-poches" destinée à être fixée sur la peau de l'utilisateur et munie, pour ce faire, d'un patin adhésif, et une poche de recueil propre à être assujettie par un embout qu'elle présente, de manière amovible sur cette plaque frontale.

Les appareillages pour stomie de type "deux-pièces" doivent satisfaire à des conditions dont certaines sont contradictoires entre elles. En effet, s'il est impératif que la fixation de la poche de recueil sur le porte-poches soit sûre de manière à éviter toute séparation intempestive de cette poche, notamment sous l'effet des contraintes exercées par le poids des matières corporelles dont elle se remplit, et garantisse par ailleurs une parfaite étanchéité de l'appareillage vis-à-vis de ces matières, il convient également que les stomisés - qui sont souvent des personnes âgées - puissent, après une période d'apprentissage par le personnel soignant, procéder eux-mêmes à la mise en place d'une part, du porte-poches et, d'autre part, des poches de recueil sur ce dernier, ce qui implique que les manipulations d'assemblage et de désassemblage de la poche et du porte-poches soient aussi simples et aisées que possible.

A cet effet, on a proposé, dans de nombreuses formes de réalisation, des appareillages deux-pièces dans lesquels l'assemblage de la poche de recueil et du porte-poches est réalisé par l'emboîtement à force, sous l'effet d'une pression appliquée par le patient, d'un embout qui est muni d'une lèvre annulaire d'étanchéité et qui est situé de préférence sur la poche, dans un canal de formes et de dimensions conjuguées à celles dudit embout et qui est formé par deux autres embouts situés de préférence sur le porte-poches. Ces appareillages, qui sont décrits par exemple dans GB-A-1 571 657 et EP-A-0 171 255, présentent l'avantage qu'une seule opération d'emboîtement assure, d'une part, la solidarisation de la poche et du porte-poches et d'autre part, l'étanchéité de l'appareillage. Toutefois, en pratique, pour l'obtention de résultats satisfaisants, ces appareillages exigent qu'une pression importante soit exercée par le patient sur la poche lors de sa mise en place sur le porte-poches et, partant, sur la zone péristomiale, laquelle est généralement très sensible, parfois même douloureuse, en sorte que l'utilisation de ces appareillages n'est pas réellement satisfaisante.

Pour tenter de résoudre ce problème, on a proposé dans GB-A-2 237 993 et GB-A-2 237 995, d'améliorer ce type d'appareillages en conformant les embouts portés par la poche de recueil et le porte-poches de manière à ce que leur assemblage soit obtenu, non plus par un emboîtement à force, mais par l'encliquetage de l'embout de la poche entre les deux embouts du porte-poches et que leur solidarisation soit assurée par l'arc-boutement de l'extrémité de la lèvre annulaire d'étanchéité portée par l'embout de la poche contre une nervure, également annulaire, située sur l'un des embouts du porte-poches. Si ces appareillages permettent effectivement d'assembler la poche et le porte-poches sans exercer de pression sur la zone péristomiale, ils posent toutefois un autre problème qui est celui de nécessiter, pour leur désassemblage, l'application d'une force de traction importante sur ladite poche qui, là encore, peut être génératrice de douleurs compte tenu de la sensibilité de la zone péristomiale et qui peut provoquer, de plus, un arrachement partiel ou total du porte-poches de la peau du patient le rendant impropre à être utilisé plus longtemps.

On a, par ailleurs, proposé des appareillages deux-pièces dans lesquels le dispositif d'assemblage de la poche de recueil et du porte-poches comprend, d'une part, deux embouts dont l'un est situé sur la poche tandis que l'autre est fixé sur le porte-poches et qui sont prévus pour assurer, lorsqu'ils sont mis en contact l'un de l'autre, l'étanchéité de l'appareillage grâce à la présence d'une lèvre ou d'un joint d'étanchéité ou à la conjugaison de leurs formes et/ou de leurs dimensions et, d'autre part, un organe du type bague circulaire qui est généralement associé à l'un de ces deux embouts et qui assure, par différents mécanismes (effet de came, effet de serrage, ...) la solidarisation de la poche sur le porte-poches, de manière à ce que l'assemblage et le désassemblage de ces derniers puissent être réalisés en exerçant sur la poche des forces de pression dans un cas et de traction dans l'autre modérées, voire inexistantes. A titres d'exemples, de tels appareillages sont décrits dans EP-A-0 255 310 et dans WO-A-91/01118.

Les appareillages de ce dernier type ne donnent pas non plus totalement satisfaction, dans la mesure où, bien qu'appartenant au type des appareillages deux-pièces, ils comprennent en réalité, pour l'assemblage de la poche de recueil sur le porte-poches, au moins trois éléments, ce qui complique notablement leur réalisation, tant au niveau de la fabrication des différentes pièces qui les constituent que de l'assemblage de ces pièces, et augmente substantiellement leur coût de production, alors que l'utilisation au long cours d'appareillages par les stomisés justifie que ces appareillages soient relativement peu onéreux.

C'est, par conséquent, un but général de l'Invention que de fournir un appareillage pour stomie dont le dispositif assurant l'assemblage et le désassemblage de la poche de recueil et du porte-poches ne soit constitué que de deux éléments de manière à présenter un coût de production peu élevé, tout en satisfaisant à toutes les conditions requises pour un tel appareillage et, notamment de simplicité d'utilisation, de maniabilité, de sécurité de fixation de la poche sur le porte-poches et d'étanchéité vis-à-vis des matières corporelles et qui, non seulement, permette une mise en place de la poche sur le porte-poches sans application de pression au niveau de la zone péristomiale, mais autorise également un retrait de cette poche sans qu'il soit nécessaire d'exercer une force de traction importante.

C'est, également, un but de l'Invention de fournir un tel appareillage qui permette au patient, une fois la poche de recueil et le porte-poches assemblés, de modifier facilement et sans risque le positionnement de la poche par rapport au porte-poches, par exemple pour adapter l'orientation de la poche en fonction de ses activités ou pour en faciliter la vidange.

Ces buts sont atteints selon l'Invention par un raccord propre à entrer dans la constitution d'un appareillage pour stomie et qui comprend deux éléments propres à être assemblés de manière amovible et comportant chacun une embase munie d'une ouverture centrale et un embout tubulaire entourant ladite ouverture, dans lequel l'embout du premier élément comprend sur sa face interne une lèvre annulaire à déformation élastique dont l'extrémité libre est dirigée vers le plan de l'embase dudit élément tandis que l'embout du deuxième élément comporte sur sa face externe des moyens de retenue axiale de l'extrémité libre de cette lèvre, lequel raccord est caractérisé en ce qu'il comporte un organe de verrouillage et en ce que les moyens de retenue axiale de l'extrémité libre de la lèvre annulaire occupent un secteur de la circonférence de l'embout du deuxième élément dont le point médian se trouve diamétralement opposé audit organe de verrouillage en conditions d'assemblage et de désassemblage des deux éléments de ce raccord.

Ainsi, pour assembler les deux éléments du raccord, l'utilisateur, après s'être assuré que l'organe de verrouillage est en position de déverrouillage, commence par positionner la lèvre annulaire du premier élément en appui sur les moyens de retenue axiale du deuxième élément dans la zone diamétralement opposée à l'organe de verrouillage. Puis, il fait pivoter un élément par rapport à l'autre jusqu'à ce que les embouts des deux éléments soient engagés à fond l'un dans l'autre. Il lui suffit alors de verrouiller l'organe de verrouillage pour obtenir la solidarisation des deux éléments du raccord. Cet assemblage ne nécessite donc l'application d'aucune pression.

Pour désassembler les deux éléments du raccord, il suffit d'effectuer une opération inverse : déverrouiller l'organe de verrouillage, écarter l'un de l'autre les deux éléments du raccord au droit de l'organe de verrouillage de manière à faire subir un pivotement à l'un des éléments du raccord par rapport à l'autre, la lèvre annulaire demeurant en appui sur les moyens de retenue dans la zone diamétralement opposée à l'organe de verrouillage ; ainsi, avec le pivotement, la lèvre peut finalement être dégagée complètement par simple effet de géométrie et, donc, sans qu'il soit nécessaire d'exercer des tractions.

Selon une disposition avantageuse du raccord conforme à l'Invention, les moyens de retenue axiale de l'extrémité libre de la lèvre annulaire occupent un secteur de la circonférence de l'embout du deuxième élément correspondant à un angle compris entre environ 180 et 320° selon le diamètre de cet embout, lequel est lui même avantageusement compris entre environ 25 et 100 mm. Ainsi, dans le cas d'un embout présentant un diamètre inférieur ou sensiblement égal à 50 mm, les moyens de retenue axiale de l'extrémité de la lèvre annulaire occuperont de préférence un secteur angulaire compris entre 180 et 275°, tandis que dans le cas d'un embout d'un diamètre plus élevé, ils s'étendront de préférence sur un secteur angulaire compris entre 240 et 320°.

Selon une disposition préférée du raccord selon l'Invention, les moyens de retenue axiale de l'extrémité libre de la lèvre annulaire sont constitués par un rebord s'étendant radialement, à partir de la face externe de l'embout du deuxième élément, vers l'extérieur de cet embout.

De manière particulièrement préférée, ce rebord est un rebord mourant dont la largeur est maximale au point médian du secteur qu'il occupe et diminue progressivement en direction des extrémités de ce secteur.

Pour garantir le verrouillage des deux éléments du raccord, ce dernier comprend avantageusement des moyens d'immobilisation de l'organe de verrouillage en condition de verrouillage.

Selon une autre disposition préférée du raccord selon l'invention, l'organe de verrouillage étant associé à l'un des deux éléments, il est prévu pour coopérer, en réponse à l'actionnement d'un mécanisme de commande associé au même élément que lui, avec une surface d'appui ménagée sur l'embout de l'autre des deux éléments de ce raccord.

Selon une première forme de réalisation préférée du raccord conforme à l'Invention, l'organe de verrouillage et le mécanisme de commande sont associés au deuxième élément de ce raccord et l'organe de verrouillage est prévu pour coopérer avec une surface d'appui s'étendant radialement, à partir de la face externe de l'embout du premier élément, vers l'extérieur de cet embout.

Selon une modalité avantageuse de cette première forme de réalisation préférée, la surface d'appui destinée à coopérer avec l'organe de verrouillage est formée par le pan faisant face à l'embase du premier élément d'une nervure à pans coupés en saillie sur la face externe de l'embout de ce premier élément.

De manière préférée, la nervure à pans coupés en saillie sur la face externe de l'embout du premier élément s'étend sur toute la circonférence de cet embout. Ainsi, en condition d'utilisation de l'appareillage pour stomie, le verrouillage des deux éléments du raccord est assuré quelle que soit la position de la poche de recueil par rapport au porte-poches, en sorte que le patient peut modifier à volonté cette position, par rotation de ladite poche sur le porte-poches, sans risque de provoquer un déverrouillage desdits éléments.

Selon une autre modalité avantageuse de cette première forme de réalisation préférée, l'organe de verrouillage est d'un seul tenant avec le mécanisme de commande et est constitué par au moins une nervure à pans coupés dont l'un des pans est conjugué au pan de la nervure de l'embout du premier élément destiné à servir de surface d'appui, et qui fait saillie sur la face dudit mécanisme de commande située en regard de l'embout du deuxième élément.

De manière préférée, le mécanisme de commande comprend un bras monté à pivotement autour d'un axe formé par un tourillon qu'il présente et qui est engagé dans un orifice ménagé dans l'embase du deuxième élément.

Dans ce cas, le deuxième élément comporte avantageusement des moyens pour maintenir et guider ce bras dans un plan parallèle à celui de son embase.

Avantageusement, ces moyens de maintien et de guidage du bras comprennent au moins un ergot faisant saillie sur la face de ce bras située en regard de l'embase du deuxième élément, présentant une extrémité libre à retour déterminant une face d'appui et adaptée à traverser une boutonnière ménagée dans l'embase du deuxième élément, de sorte que ladite face d'appui glisse sur une face de ladite embase située du côté opposé audit bras.

Dans ce cas également, le bras comporte avantageusement, sur sa face située en regard de l'embase du deuxième élément, au moins une pièce saillante adaptée à coopérer avec un bec ménagé sur le bord externe de cette même embase pour assurer le blocage de l'organe de verrouillage en condition de verrouillage.

Selon encore une autre modalité avantageuse de cette première forme de réalisation préférée, le deuxième élément du raccord comporte, de plus, des moyens propres à limiter le déplacement radial de la base de la lèvre annulaire en direction de l'embout du premier élément lorsque les deux éléments de ce raccord sont assemblés.

Selon une autre forme de réalisation préférée du raccord conforme à l'Invention, l'organe de verrouillage est associé au premier élément de ce raccord et est prévu pour coopérer, en réponse à l'actionnement d'un mécanisme de commande également associé audit premier élément, avec une surface d'appui s'étendant radialement, à partir de la face externe de l'embout du deuxième élément, en direction de l'intérieur de cet embout.

Selon une modalité avantageuse de cette forme de réalisation préférée, la surface d'appui destinée à coopérer avec l'organe de verrouillage est formée par la paroi située en regard de l'embase du deuxième élément d'un évidement ménagé dans l'épaisseur de l'embout de ce deuxième élément.

De manière préférée, l'évidement ménagé dans l'épaisseur de l'embout du deuxième élément s'étend sur toute la circonférence de cet embout. Toutefois, dans la mesure où, dans cette forme de réalisation, l'organe de verrouillage est associé au premier élément du raccord tandis que les moyens de retenue axiale de l'extrémité de la lèvre annulaire de ce premier élément se situent sur le deuxième élément de ce raccord, il est avantageux de prévoir sur l'embase du deuxième élément la présence de deux plots propres à limiter la rotation d'un élément par rapport à l'autre lorsque ces éléments sont assemblés, de manière à éviter que l'organe de verrouillage ne soit amené à coopérer avec une portion de cet évidement qui serait située dans le secteur angulaire occupé par lesdits moyens de retenue axiale, créant ainsi une zone au niveau de laquelle les embouts des deux éléments du raccord seraient dépourvus à la fois de moyens propres à assurer leur verrouillage et de moyens propres à retenir axialement l'extrémité libre de la lèvre annulaire, et présentant une extension angulaire suffisante pour entraîner une désolidarisation involontaire de ces deux éléments.

Selon une autre modalité avantageuse de cette forme de réalisation préférée, l'organe de verrouillage est d'un seul tenant avec le mécanisme d'actionnement et est constitué par une tige qui est placée dans une ouverture cylindrique traversant l'embout du premier élément, dont une extrémité est propre à être logée dans l'évidement ménagé dans l'épaisseur de l'embout du deuxième élément tandis que son autre extrémité est solidaire du mécanisme d'actionnement.

De manière préférée, cette tige est filetée sur tout ou partie de sa longueur et le mécanisme d'actionnement est formé par une molette ou analogue propre à permettre son déplacement par rotation dans l'ouverture cylindrique dans laquelle elle est placée.

L'Invention a également pour objet un appareillage pour stomie tel qu'un appareillage pour iléostomie, colostomie, urostomie ou pour le drainage post-chirurgical d'espaces intra-abdominaux du type drainage péritonéal ou sous-péritonéal, qui comprend un porte-poches destiné à être fixé autour d'une ouverture artificielle du corps d'un utilisateur, ainsi qu'une poche pour le recueil des matières corporelles propres à être assemblée de manière amovible audit porte-poches, caractérisé en ce qu'il comprend un raccord tel que défini ci-dessus.

Outre les dispositions qui précèdent, l'Invention comprend encore d'autres dispositions qui ressortiront de la description qui suit, faite à titre d'exemple et en référence aux dessins annexés dans lesquels :
- la Figure 1a est une vue en plan du premier élément d'un raccord pour appareillage de stomie selon l'Invention pour une première forme de réalisation de ce raccord ;
- la Figure 1b est une vue en coupe, à plus grande échelle, selon la ligne 1b-1b de la Figure 1a ;
- la Figure 2 est une vue en plan du deuxième élément d'un raccord pour appareillage pour stomie selon l'Invention pour cette première forme de réalisation du raccord ;
- la Figure 3 est une vue en perspective d'un élément de la Figure 2, à plus grande échelle que celle de la Figure 2 ;
- la Figure 4 est une vue en perspective d'un détail de l'élément de la Figure 2, à plus grande échelle que celle de la Figure 2 ;
- la Figure 5 est une vue arrière en perspective du détail de la Figure 4 ;
- la Figure 6 est une vue en coupe, à plus grande échelle, selon la ligne 6-6 de la Figure 2 ;
- la Figure 7 est une vue en coupe, à plus grande échelle, selon la ligne 7-7 de la Figure 2 ;
- la Figure 8 est une vue schématique en coupe selon la ligne 8-8 de la Figure 2 après assemblage de l'élément de la Figure 1 et de l'élément de la Figure 2, à plus grande échelle que celles de ces Figures ; et
- la Figure 9 est une vue schématique en coupe analogue à celle de la Figure 8, mais pour une deuxième forme de réalisation d'un raccord pour appareillage pour stomie selon l'Invention.

Il doit être bien entendu, toutefois, que ces dessins et les parties descriptives correspondantes sont donnés uniquement à titre d'illustration de l'objet de l'Invention et n'en constituent aucunement une limitation.

Sur les dessins, les éléments des différentes formes de réalisation qui sont équivalents ont reçu des numéros de référence identiques.

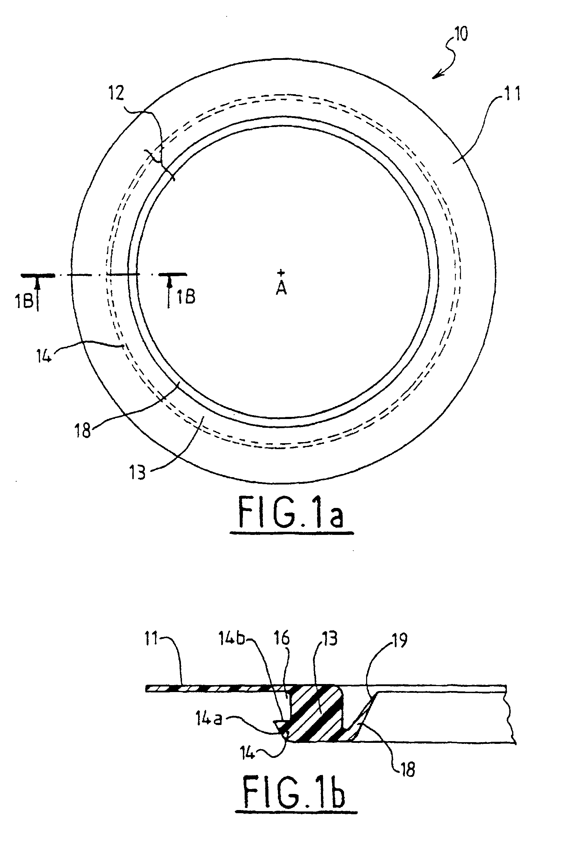

On se réfère tout d'abord aux Figures 1 à 8 qui montrent une première forme de réalisation d'un raccord pour appareillage pour stomie conforme à l'Invention et qui comprend deux éléments 10 et 20 destinés à être fixés pour le premier, sur la poche de recueil et pour le second, sur le porte-poches d'un tel appareillage, et adaptés à être assemblés de manière amovible comme il sera explicité ci-après.

Le premier élément du raccord - qui est représenté sur les Figures 1a et 1b - ou élément 10 comprend une embase annulaire 11, d'axe de révolution A, et sur le bord interne de laquelle est érigé un embout tubulaire 12. Cet embout comprend une paroi cylindrique 13 qui est perpendiculaire à l'embase 11 et de même axe qu'elle, et qui comporte en saillie sur sa face externe une nervure annulaire 14 à deux pans coupés 14a et 14b, cette nervure délimitant avec ladite embase une gorge annulaire 16. La paroi cylindrique 13 comporte, par ailleurs, en saillie sur sa face interne une lèvre annulaire 18 à déformation élastique qui s'étend obliquement de l'extrémité libre de cette paroi en direction du plan de l'embase 11 et qui est destinée à assurer l'étanchéité du raccord vis-à-vis des matières corporelles recueillies en condition d'utilisation de l'appareillage pour stomie.

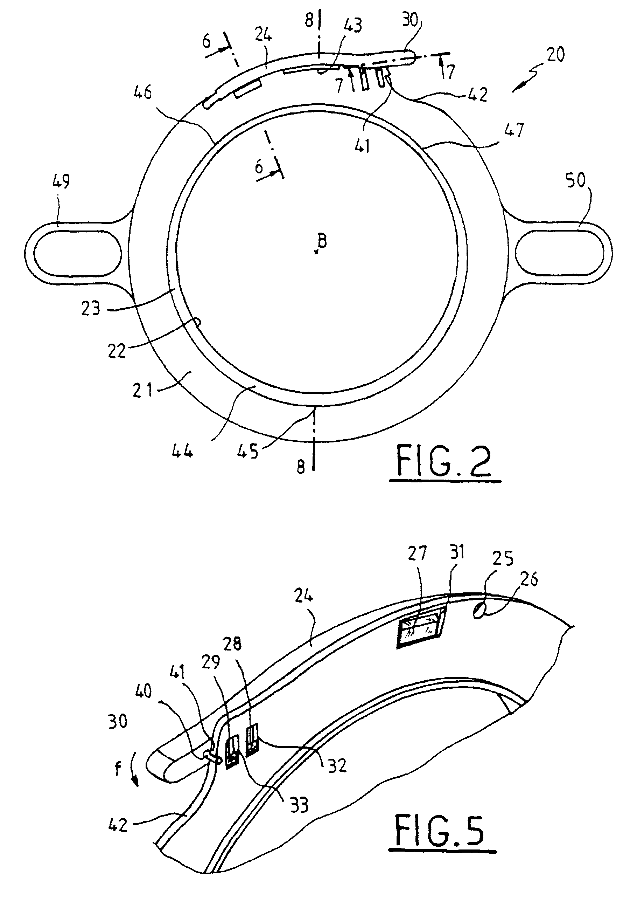

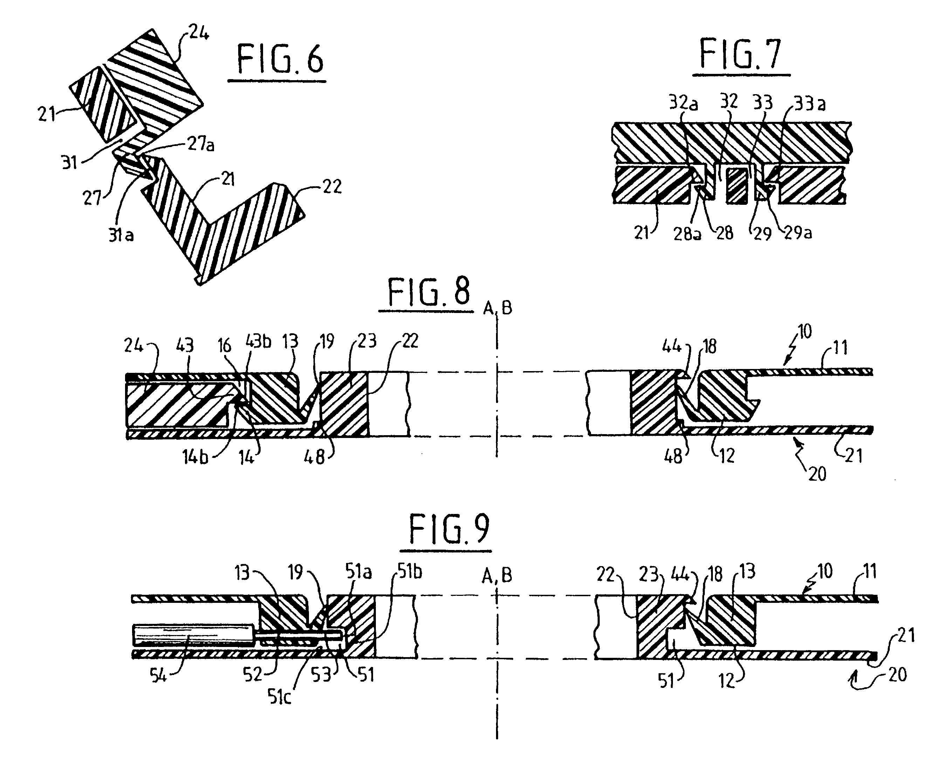

Le deuxième élément du raccord ou élément 20 - qui est, lui, représenté sur les Figures 2 à 7 - comprend également une embase annulaire 21, d'axe de révolution B, et qui porte, sur son bord interne, un embout tubulaire 22 comprenant une paroi cylindrique 23 perpendiculaire à ladite embase et coaxiale avec cette dernière. Cet embout est prévu pour être emboîté dans l'embout 12 de l'élément 10, lors de l'assemblage des deux éléments 10 et 20 du raccord, et est conformé de manière à ce qu'au cours de cet emboîtement l'extrémité libre 19 de la lèvre annulaire 18 de l'embout 12 vienne, comme visible sur la Figure 8, s'appliquer contre la face externe de ladite paroi cylindrique 23 et puisse ainsi remplir sa fonction d'organe d'étanchéité. De ce fait, le diamètre externe de la paroi cylindrique 23 de l'embout 22 est choisi de manière à être légèrement inférieur au diamètre interne de la paroi cylindrique 13 de l'embout 12, tout en étant légèrement supérieur au diamètre interne que présente la lèvre annulaire 18 de l'embout 12 au niveau de son extrémité libre 19, lorsque les deux embouts 12 et 22 ne sont pas emboîtés.

L'élément 20 est muni d'un bras 24 de forme générale curviligne et à section droite rectangulaire, qui - comme visible sur la Figure 5 - est monté à pivotement par un tourillon 25 qu'il présente dans un orifice 26 ménagé dans l'embase 21 à faible distance du bord externe de cette dernière et dont l'actionnement est propre à assurer, par l'intermédiaire d'une nervure qu'il porte et qui sera décrite ci-après, le verrouillage et le déverrouillage des deux éléments 10 et 20 du raccord.

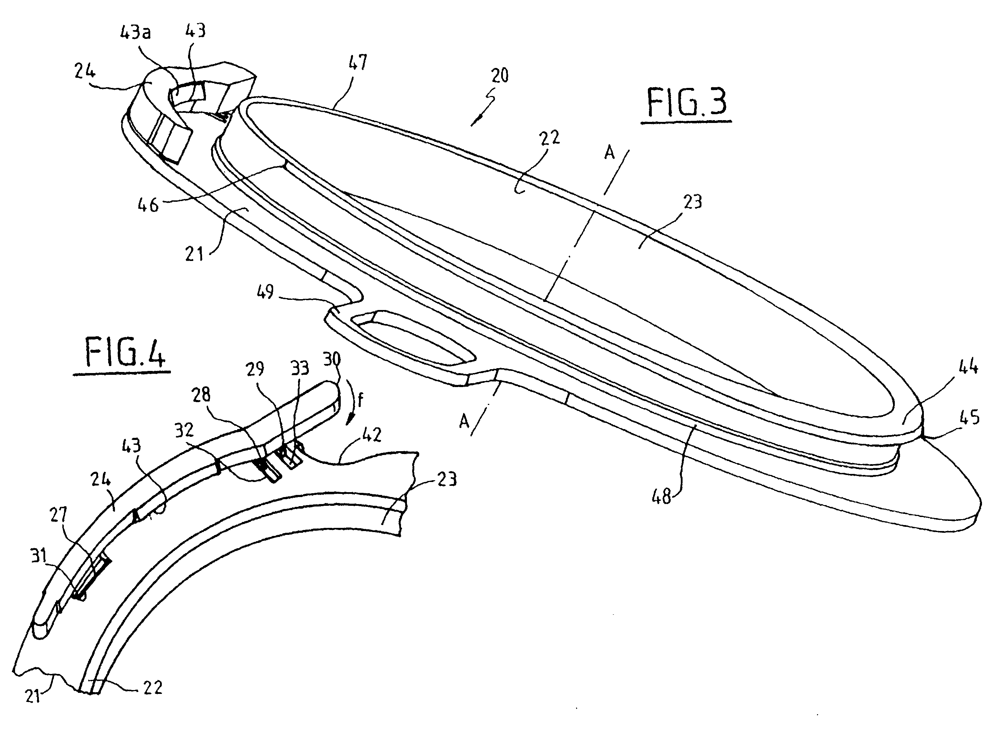

Ce bras 24 comporte, sur sa face située en regard de l'embase 21, trois ergots, respectivement 27, 28 et 29, disposés pour le premier, à proximité du tourillon 25, et pour les deux autres, à proximité de l'extrémité libre 30 de ce bras et qui sont logés chacun dans une boutonnière de forme conjuguée, respectivement 31, .32 et 33, ménagée dans l'embase 21. Ces ergots 27, 28 et 29 ont pour fonction de maintenir le bras 24 dans un plan parallèle à celui de l'embase 21 lors de son pivotement, et d'en assurer le guidage, notamment en limitant l'amplitude de ce pivotement dans ce même plan entre une position de déverrouillage qui est celle représentée sur les Figures 2 et 4 et une position de verrouillage qui est celle montrée sur la Figure 5. Pour ce faire, comme visible sur les Figures 6 et 7, chacun des ergots 27, 28 et 29 présente à son extrémité libre une face , respectivement 27a, 28a et 29a, d'appui glissant sur une face, respectivement 31a, 32a et 33a, disposée en vis-à-vis sur l'une des parois de chacune des boutonnières 31, 32 et 33. Comme illustré sur les Figures 6 et 7, les faces 27a, 28a, 29a, 31a, 32a et 33a sont avantageusement des faces planes.

Par ailleurs, comme visible sur la Figure 5, le bras 24 présente, toujours sur sa face située en regard de l'embase 21 et entre l'ergot 29 et l'extrémité libre 30 de ce bras, un téton cylindrique 40 propre à assurer son blocage en position de verrouillage après franchissement dans le sens de la flèche f d'un bec 41 qui est situé sur le bord externe de l'embase 21, à l'extrémité d'une portion de cette embase conformée suivant une anse 42 de manière à faciliter la préhension de ladite extrémité libre 30 de ce bras.

Ainsi que précédemment mentionné, le bras 24 présente, de plus, en saillie sur la partie médiane de sa face située en regard de l'embout 22, une nervure 43 qui est, comme la nervure annulaire 14 de l'embout 12, à deux pans coupés 43a et 43b et dont le pan 43b est conjugué au pan 14b de cette nervure annulaire de manière à assurer, après emboîtement de l'embout 22 dans l'embout 12 et positionnement du bras 24 en position de verrouillage, le verrouillage de l'élément 10 sur l'élément 20 par coopération avec une portion de même longueur qu'elle de ladite nervure annulaire 14.

Comme bien visible sur la Figure 3, la paroi cylindrique 23 de l'embout 22 porte, sur sa face externe et au niveau de son extrémité libre, un rebord 44 qui est sensiblement parallèle à l'embase 21 et qui s'étend radialement en direction du bord externe de cette embase (extension qui a été accentuée pour des raisons de visibilité sur la Figure 3).

Conformément à l'Invention, ce rebord 44, dont la fonction est de retenir axialement l'extrémité libre 19 de la lèvre annulaire 18 de l'embout 12, lorsque les embouts 12 et 22 sont emboîtés, n'est pas présent sur la totalité de la circonférence de l'embout 22, mais occupe un secteur de cette circonférence correspondant à un angle sensiblement égal à 250° et dont le point médian 45 se trouve diamétralement opposé au milieu de la nervure 43 portée par le bras 24.

Par ailleurs, ce rebord 44 est un rebord mourant dont la largeur est maximale au point médian 45 du secteur circulaire qu'il occupe - au niveau duquel cette largeur est avantageusement comprise entre 0,5 et 2 mm selon le diamètre de l'embout 22 (qui est lui même avantageusement compris entre 25 et 100 mm) - et diminue progressivement en direction des extrémités 46 et 47 de ce secteur.

Comme visible sur les Figures 3 et 8, la paroi cylindrique 23 de l'embout 22 comporte, de plus, en saillie sur sa face externe un bourrelet annulaire 48 à section droite sensiblement rectangulaire. Ce bourrelet annulaire est destiné à servir, en condition d'utilisation de l'appareillage pour stomie, de butée vis-à-vis de la base de la lèvre annulaire 18 de l'embout 12 de manière à éviter que cette dernière, sous l'effet du poids des matières corporelles recueillies dans la poche de recueil, ne vienne s'écraser contre la paroi cylindrique 23 de l'embout 22 et que, du fait de cet écrasement, l'extrémité libre 19 de cette lèvre ne s'écarte de cette paroi, entraînant ainsi une perte d'étanchéité, voire ne se dégage du rebord 44, entraînant alors la désolidarisation de la poche et du porte-poches.

Le fonctionnement de cette première forme de réalisation d'un raccord conforme à l'Invention résulte immédiatement de ce qui précède. Pour l'assemblage des éléments 10 et 20, on présente, après s'être assuré au préalable que le bras 24 est en position de déverrouillage, ces deux éléments en regard l'un de l'autre et on les positionne de manière à mettre la lèvre annulaire 18 portée par l'embout 12 de l'élément 10 en appui sur le rebord 44 de l'embout 22 de l'élément, au niveau du point médian de ce rebord. Puis, on procède à l'emboîtement de l'embout 22 dans l'embout 12 en faisant pivoter un élément par rapport à l'autre. Compte tenu de l'aptitude de la lèvre annulaire 18 à être déformée élastiquement, cet emboîtement s'effectue pratiquement sans application de pression et ce, bien que l'extrémité libre 19 de cette lèvre présente, comme précédemment mentionné, un diamètre interne plus faible que le diamètre externe de la paroi cylindrique 23 de l'embout 22 et ait à franchir, sur une partie de sa circonférence, le rebord 44 porté par cette paroi avant de s'appliquer contre la face externe de cette dernière. Lorsque l'embout 22 est engagé à fond dans l'embout 12, on pivote alors le bras 24 dans le sens de la flèche f jusqu'à ce que le téton 40 franchisse le bec 41 et se trouve bloqué par ce dernier, ce qui a pour effet, comme illustré sur la Figure 8, de faire pénétrer la nervure 43 de ce bras dans la portion de la gorge annulaire 16 qui se trouve en vis-à-vis d'elle, de manière à ce que l'appui, sur cette nervure, de la portion de la nervure annulaire 14 de l'embout 12 qui limite cette portion de gorge, s'oppose à toute séparation des embouts 12 et 22.

Ainsi, dans cette condition, la solidarisation des deux éléments 10 et 20 du raccord est assurée d'une part, sur la partie de la circonférence de l'embout 22 où le rebord 44 est présent, par ce rebord qui garantit le blocage mécanique de l'extrémité libre 19 de la lèvre annulaire 18 de l'embout 12 dans le cas où celle-ci serait amenée à se déplacer axialement (par exemple, en condition d'utilisation de l'appareillage pour stomie, sous l'effet du poids des matières recueillies dans la poche de recueil) et, d'autre part, sur la partie de la circonférence de l'embout 22 où le rebord 44 est absent, par la nervure 43 portée par le bras 24 qui, par coopération avec une portion de même longueur qu'elle de la nervure annulaire 14 de l'embout 12, garantit le verrouillage desdits éléments 10 et 20.

Dans cette condition toutefois, il est possible de déplacer un élément du raccord par rapport à l'autre, non pas à l'extraction, mais par une rotation autour de son axe, notamment pour modifier, en condition d'utilisation de l'appareillage pour stomie, le positionnement de la poche par rapport au porte-poches et ce, sans aucun risque de provoquer une séparation involontaire de ces éléments.

Pour séparer les deux éléments 10 et 20 du raccord, il convient de faire échapper le téton 40 du bec 41, de pivoter le bras 24 dans le sens inverse de celui montré par la flèche f, ce qui permet de dégager la nervure 43 de la portion de la gorge annulaire 16 de l'embout 12 dans laquelle elle se trouvait et de libérer ainsi la portion de la nervure annulaire 14 avec laquelle elle coopérait, puis de tirer légèrement sur l'embase de l'un des deux éléments, par exemple sur la portion d'embase 11 de l'élément 10 qui est située en regard de l'anse 42 ménagée dans l'embase 21 de l'élément 20, de manière à provoquer le désemboîtement des embouts 12 et 22 au droit de la nervure 43. Ce désemboîtement est obtenu sans difficulté puisqu'au niveau de cette nervure, l'embout 22 est dépourvu de rebord susceptible de retenir axialement l'extrémité libre 19 de la lèvre annulaire 18 de l'embout 12. Il suffit alors de pivoter un élément par rapport à l'autre pour libérer l'extrémité libre 19 de la lèvre annulaire 18 du rebord 44 et obtenir, ainsi, la séparation des deux éléments du raccord.

Le fonctionnement de ce raccord est donc très simple et sûr et ne nécessite l'application, que ce soit pour l'assemblage des deux éléments de ce raccord, comme pour leur désassemblage, que des forces (de pression dans le premier cas, et de traction dans le deuxième cas) extrêmement modérées.

Comme précédemment mentionné, les éléments 10 et 20 de la forme de réalisation du raccord conforme à l'Invention qui vient d'être décrite, sont destinés à être fixés respectivement sur la poche de recueil et sur le porte-poches d'un appareillage pour stomie. C'est la raison pour laquelle, comme visible sur la Figure 2, sont prévues, sur le bord externe de l'embase 21 de l'élément 20, deux oreilles 49 et 50 qui sont diamétralement opposées et qui saillent radialement vers l'extérieur de cette embase. Ces oreilles sont percées chacune d'un orifice et permettent avantageusement, si le patient le souhaite, de renforcer le maintien du porte-poches sur la zone péristomiale au moyen d'une ceinture.

La fixation de l'élément 10 sur la poche de recueil peut être réalisée en soudant ou en collant directement l'embase 11 de cet élément autour de l'ouverture ménagée dans l'une des parois de cette poche pour permettre l'écoulement dans ladite poche des matières corporelles évacuées par la stomie au travers du porte-poches, tandis que la fixation de l'élément 20 sur le porte-poches est, de préférence, réalisée, de manière connue en soi, par l'intermédiaire d'une collerette circulaire (non représentée sur les Figures 2 à 7) s'étendant radialement du bord interne de l'embase 21 en direction du centre de cette dernière, par soudure, collage ou tout autre moyen, de cette collerette sur la face dudit porte-poches opposée à celle destinée à être fixée sur le corps du patient, autour de l'ouverture prévue pour entourer, en condition d'utilisation, la stomie et de manière à ce que le bras 24 soit placé à la partie supérieure de ce porte-poches.

En variante, il est, toutefois, possible de prévoir un appareillage pour stomie dans lequel, à l'inverse, ce serait le porte-poches qui serait muni de l'élément 10 et la poche de recueil qui comprendrait l'élément 20, en sorte que dans ce cas les oreilles 49 et 50 seraient présentes sur le bord externe de l'embase 11 de l'élément 10. Il est, bien entendu, également possible de prévoir un appareillage dans lequel ni l'élément 10, ni l'élément 20 ne seraient munis d'oreilles.

La Figure 9 illustre une autre forme de réalisation d'un raccord conforme à l'Invention dans laquelle l'organe de verrouillage et son mécanisme de commande sont associés au premier élément 10 et non, au deuxième élément 20 de ce raccord comme dans la forme de réalisation précédemment décrite.

De ce fait, dans cette forme de réalisation, la paroi cylindrique 13 de l'embout 12 du premier élément, si elle comprend, comme dans la forme de réalisation précédemment décrite, en saillie sur sa face interne une lèvre annulaire 18 à déformation élastique destinée à assurer l'étanchéité du raccord vis-à-vis des matières corporelles recueillies en condition d'utilisation de l'appareillage de stomie, est dépourvue de nervure annulaire sur sa face externe. Par contre, la paroi cylindrique 23 de l'embout 22 du deuxième élément comporte une gorge annulaire 51 qui s'étend radialement dans son épaisseur à partir de sa face externe et qui est délimitée par trois parois : une première paroi 51a sensiblement parallèle à l'embase 21, une deuxième paroi 51b sensiblement perpendiculaire à cette embase et qui constitue le fond de cette gorge et une troisième paroi formée par l'embase 21 elle-même.

Par ailleurs, la paroi cylindrique 13 de l'embout 12 du premier élément 10 est traversée au niveau de la base de la lèvre annulaire 18 par une ouverture cylindrique 52 avantageusement taraudée, dans laquelle est disposée une tige cylindrique filetée 53 conformée de manière à ce que son extrémité interne - c'est-à-dire l'extrémité de cette tige qui émerge de la paroi cylindrique 23 - est conformée de manière à pouvoir pénétrer dans la gorge annulaire 51 de l'embout 22. L'extrémité externe de la tige filetée 53 - c'est-à-dire l'extrémité qui émerge de la face externe de la paroi cylindrique 13 - est, quant à elle, munie d'une molette 54 propre à permettre, après emboîtement de l'embout 22 dans l'embout 12, le déplacement par rotation de ladite tige dans l'ouverture 52 entre une position de déverrouillage et une position de verrouillage qui est celle montrée sur la Figure 9 et dans laquelle son extrémité interne est logée dans une portion de la gorge annulaire 51 de manière à ce que l'appui, sur la portion de la paroi 51a délimitant cette portion de gorge, de la tige filetée 53 s'oppose à toute séparation des embouts 12 et 22.

Ainsi, comme visible sur la Figure 9, dans cette condition, la solidarisation des deux éléments 10 et 20 du raccord est assurée, d'une part, sur la partie de la circonférence de l'embout 22 où le rebord 44 est présent, par ce rebord qui garantit le blocage mécanique de l'extrémité libre 19 de la lèvre annulaire 18 de l'embout 12 dans le cas où cette dernière serait amenée à se déplacer axialement et, d'autre part, sur la partie de la circonférence de l'embout 22 où le rebord 44 est absent, par l'extrémité interne de la tige filetée 53 portée par l'embout 12 qui, par coopération avec une portion de la gorge annulaire 51 de l'embout 22, garantit le verrouillage desdits éléments 10 et 20.

Dans ce cas également, il est possible de déplacer à rotation un élément du raccord par rapport à l'autre pour modifier, en condition d'utilisation de l'appareillage pour stomie, le positionnement de la poche par rapport au porte-poches. Toutefois, il est avantageux de prévoir, dans cette forme de réalisation, la présence sur l'embase de deux plots propres à limiter la rotation d'un élément par rapport à l'autre sur un secteur angulaire prédéterminé lorsque ces éléments sont assemblés, de manière à éviter que la tige filetée 53 ne soit amenée à coopérer avec une portion de la gorge annulaire 51 qui serait située dans le secteur angulaire occupé par le rebord 44 de l'embout 22, et qu'il ne se crée ainsi une zone au niveau de laquelle les embouts 12 et 22 seraient dépourvus à la fois de moyens propres à retenir axialement l'extrémité 19 de la lèvre annulaire 18 et de moyens propres à assurer le verrouillage des éléments 10 et 20, et présentant une extension angulaire suffisante pour entraîner une désolidarisation involontaire de ces deux éléments.

Ainsi, dans le cas où le rebord 44 occupe un secteur angulaire d'environ 250°, ces plots seront avantageusement disposés sur l'embase 21 de l'élément 20 de manière à limiter la rotation d'un élément par rapport à l'autre sur un secteur angulaire d'environ 90° dont le point médian est diamétralement opposé au point médian 45 du secteur angulaire occupé par ledit rebord 44.

Quelle que soit la forme de réalisation d'un raccord conforme à l'Invention, les éléments 10 et 20 sont avantageusement réalisés en une matière plastique relativement rigide (d'une dureté de préférence comprise entre 40 et 86 shore D) du type polyéthylène, polypropylène, polyamide polyester thermoplastique ou encore copolymère ABS non chargé en renfort de texture.

Ainsi que cela ressort de ce qui précède, l'Invention ne se limite nullement aux formes de réalisation qui viennent d'être décrites de façon plus explicite ; elle en embrasse au contraire toutes les variantes qui peuvent venir à l'esprit du technicien en la matière, sans s'écarter du cadre, ni de la portée de la présente Invention. Ainsi, notamment, il est possible d'utiliser, pour assurer le verrouillage des deux éléments du raccord, de nombreux autres dispositifs tant pour ce qui concerne l'organe de verrouillage que son mécanisme d'actionnement qui peut être, par exemple, constitué par un levier, un bouton poussoir ou un mécanisme à genouillère.

## Revendications

1. Raccord pour appareillage pour stomie comprenant deux éléments (10, 20) propres à être assemblés de manière amovible et comportant chacun une embase (11, 21) munie d'une ouverture centrale et un embout tubulaire (12, 22) entourant ladite ouverture, et dans lequel l'embout (12) du premier élément (10) comprend sur sa face interne une lèvre annulaire (18) à déformation élastique dont l'extrémité libre (19) est dirigée vers le plan de l'embase (11) dudit élément, tandis que l'embout (22) du deuxième élément (20) comporte sur sa face externe des moyens de retenue axiale (44) de l'extrémité libre de cette lèvre, ledit raccord étant **caractérisé en ce qu'**il comporte un organe de verrouillage (43, 53) et **en ce que** les moyens de retenue axiale de l'extrémité libre de la lèvre annulaire occupent un secteur de la circonférence de l'embout du deuxième élément dont le point médian (45) se trouve diamétralement opposé audit organe de verrouillage en conditions d'assemblage et de désassemblage des deux éléments de ce raccord.

2. Raccord selon la revendication 1, **caractérisé en ce que** les moyens de retenue axiale de l'extrémité libre de la lèvre annulaire occupent un secteur de la circonférence de l'embout du deuxième élément correspondant à un angle compris entre environ 180 à 320°.

3. Raccord selon la revendication 1 ou la revendication 2, **caractérisé en ce que** les moyens de retenue axiale de l'extrémité libre de la lèvre annulaire sont constitués par un rebord (44) s'étendant radialement, à partir de la face externe de l'embout du deuxième élément, vers l'extérieur de cet embout.

4. Raccord selon la revendication 3, **caractérisé en ce que** le rebord est un rebord mourant dont la largeur est maximale au point médian (45) du secteur qu'il occupe et diminue progressivement en direction des extrémités (46, 47) de ce secteur.

5. Raccord selon l'une quelconque des revendications 1 à 4, **caractérisé en ce qu'**il comprend des moyens de blocage de l'organe de verrouillage en condition de verrouillage.

6. Raccord selon l'une quelconque des revendications 1 à 5, **caractérisé en ce que** l'organe de verrouillage (43, 53) étant associé à l'un des deux éléments du raccord, il est prévu pour coopérer, en réponse à l'actionnement d'un mécanisme de commande (24, 54) associé au même élément que lui, avec une surface d'appui ménagée sur l'embout de l'autre des deux éléments du raccord.

7. Raccord selon la revendication 6, **caractérisé en ce que** l'organe de verrouillage (43) et le mécanisme de commande (24) sont associés au deuxième élément et **en ce que** l'organe de verrouillage est prévu pour coopérer avec une surface d'appui (14b) s'étendant radialement à partir de la face externe de l'embout du premier élément vers l'extérieur de cet embout.

8. Raccord selon la revendication 7, **caractérisé en ce que** la surface d'appui destinée à coopérer avec l'organe de verrouillage est formée par le pan (14b) situé en regard de l'embase du premier élément d'une nervure (14) à pans coupés (14a, 14b) faisant saillie sur la face externe de l'embout de ce premier élément.

9. Raccord selon la revendication 7, **caractérisé en ce que** la nervure à pans coupés faisant saillie sur la face externe de l'embout du premier élément s'étend sur toute la circonférence de cet embout.

10. Raccord selon la revendication 8 ou la revendication 9, **caractérisé en ce que** l'organe de verrouillage est d'un seul tenant avec le mécanisme de commande et est constitué par au moins une nervure (43) à pans coupés (43a, 43b), dont l'un des pans (43b) est conjugué au pan de la nervure de l'embout du premier élément destiné à servir de surface d'appui, et qui fait saillie sur la face dudit mécanisme de commande située en regard de l'embout du deuxième élément.

11. Raccord selon l'une quelconque des revendications 6 à 10, **caractérisé en ce que** le mécanisme de commande comprend un bras (24) monté à pivotement autour d'un axe formé par un tourillon (25) qu'il présente et qui est engagé dans un orifice (26) ménagé dans l'embase du deuxième élément.

12. Raccord selon la revendication 11, **caractérisé en ce que** le deuxième élément comporte des moyens pour maintenir et guider ce bras dans un plan parallèle à celui de son embase.

13. Raccord selon la revendication 12, **caractérisé en ce que** les moyens de maintien et de guidage du bras comprennent au moins un ergot (27, 28, 29) faisant saillie sur la face de ce bras située en regard de l'embase du deuxième élément, présentant une extrémité libre à retour déterminant une face (27a, 28a, 29a) d'appui et adaptée à traverser une boutonnière (31, 32, 33) ménagée dans l'embase du deuxième élément, de sorte que ladite face d'appui glisse sur une face (31a, 32a, 33a) de ladite embase située du côté opposé audit bras.

14. Raccord selon l'une quelconque des revendications 11 à 13, **caractérisé en ce que** ledit bras comporte, sur sa face située en regard de l'embase du deuxième élément, au moins une pièce saillante (40) adaptée à coopérer avec un bec (41) ménagé sur le bord externe de cette même embase pour assurer le blocage de l'organe de verrouillage en condition de verrouillage.

15. Raccord selon l'une quelconque des revendications 1 à 14, **caractérisé en ce que** le deuxième élément du raccord comporte, de plus, des moyens (48) propres à limiter le déplacement radial de la base de la lèvre annulaire en direction de l'embout du premier élément lorsque les deux éléments de ce raccord sont assemblés.

16. Raccord selon l'une quelconque des revendications 1 à 5, **caractérisé en ce que** l'organe de verrouillage (53) et le mécanisme de commande (54) sont associés au premier élément de ce raccord et **en ce que** l'organe de verrouillage est prévu pour coopérer avec une surface d'appui (51a) s'étendant radialement, à partir de la face externe de l'embout du deuxième élément, en direction de l'intérieur de cet embout.

17. Raccord selon la revendication 16, **caractérisé en ce que** la surface d'appui destinée à coopérer avec l'organe de verrouillage est formée par la paroi (51a) située en regard de l'embase du deuxième élément d'un évidement (51) ménagé dans l'épaisseur de l'embout de ce deuxième élément.

18. Raccord selon la revendication 17, **caractérisé en ce que** l'évidement ménagé dans l'épaisseur de l'embout du deuxième élément s'étend sur toute la circonférence de cet embout.

19. Raccord selon l'une quelconque des revendications 16 à 18, **caractérisé en ce que** l'embase du deuxième élément comporte deux plots propres à limiter la rotation d'un élément par rapport à l'autre lorsque les deux éléments de ce raccord sont assemblés.

20. Raccord selon l'une quelconque des revendications 17 à 19, **caractérisé en ce que** l'organe de verrouillage est d'un seul tenant avec le mécanisme d'actionnement et est constitué par une tige (53) qui est placée dans une ouverture cylindrique (52) traversant l'embout du premier élément, dont une extrémité est propre à être logée dans l'évidement ménagé dans l'épaisseur de l'embout du deuxième élément tandis que son autre extrémité est solidaire dudit mécanisme d'actionnement.

21. Raccord selon la revendication 20, **caractérisé en ce que** la tige est filetée sur tout ou partie de sa longueur et le mécanisme d'actionnement est formé par une molette (54) ou analogue propre à permettre son déplacement par rotation dans l'ouverture cylindrique dans laquelle elle est placée.

22. Appareillage pour stomie comprenant un porte-poches destiné à être fixé autour d'une ouverture artificielle du corps d'un utilisateur, ainsi qu'une poche pour le recueil des matières corporelles propres à être assemblée de manière amovible audit porte-poches, **caractérisé en ce qu'**il comprend un raccord selon l'une quelconque des revendications 1 à 21.

## Patentansprüche

1. Verbindungseinrichtung für eine Stomavorrichtung, mit zwei abnehmbar zusammensetzbaren Elementen (10, 20), die jeweils eine mit einer mittigen Öffnung sowie einem die Öffnung umgebenden, röhrenförmigen Ansatz (12, 22) versehene Sockelplatte (11, 12) aufweisen, wobei der Ansatz (12) des ersten Elementes (10) auf seiner Innenfläche eine elastisch verformbare, ringförmige Lippe (18) aufweist, deren freies Ende (19) auf die Ebene der Sockelplatte (11) des Elementes hin gerichtet ist, während der Ansatz (22) des zweiten Elementes (20) auf seiner Außenfläche Mittel (44) zum axialen Halten des freien Endes der Lippe aufweist, und die Verbindungseinrichtung **dadurch gekennzeichnet ist, daß** sie ein Verriegelungsorgan (43, 53) aufweist, sowie dadurch, daß die Mittel zum axialen Halten des freien Endes der ringförmigen Lippe einen Sektor des Umfangs des Ansatzes des zweiten Elementes einnehmen, dessen Medianpunkt (45) in dem Zustand beim Zusammensetzen und Abnehmen der beiden Elemente der Verbindungseinrichtung dem Verriegelungsorgan diametral gegenüberliegt.

2. Verbindungseinrichtung nach Anspruch 1, **dadurch gekennzeichnet, daß** die Mittel zum axialen Halten des freien Endes der ringförmigen Lippe einen Sektor des Umfangs des Ansatzes des zweiten Elementes einnehmen, der einem zwischen ca. 180 bis 320° betragenden Winkel entspricht.

3. Verbindungseinrichtung nach Anspruch 1 oder 2, **dadurch gekennzeichnet, daß** die Mittel zum axialen Halten des freien Endes der ringförmigen Lippe aus einer Randleiste (44) bestehen, die sich radial von der Außenfläche des Ansatzes des zweiten Elementes zur Außenseite des Ansatzes hin erstreckt.

4. Verbindungseinrichtung nach Anspruch 3, **dadurch gekennzeichnet, daß** die Randleiste eine sich verjüngende Randleiste ist, deren Breite am Medianpunkt (45) des von ihr eingenommenen Sektors am größten ist und sich in Richtung auf die Enden (46, 47) dieses Sektors hin fortlaufend verringert.

5. Verbindungseinrichtung nach einem der Ansprüche 1 bis 4, **dadurch gekennzeichnet, daß** sie Mittel zum Blockieren des Verriegelungsorgans im Verriegelungszustand aufweist.

6. Verbindungseinrichtung nach einem der Ansprüche 1 bis 5, **dadurch gekennzeichnet, daß** das Verriegelungsorgan (43, 53) einem der beiden Elemente der Verbindungseinrichtung zugeordnet und dazu vorgesehen ist, im Ansprechen auf die Betätigung eines Steuermechanismus (24, 54), der dem gleichen Element wie es selbst zugeordnet ist, mit einer Abstützfläche zusammenzuwirken, die am Ansatz des weiteren der beiden Elemente der Verbindungseinrichtung vorgesehen ist.

7. Verbindungseinrichtung nach Anspruch 6, **dadurch gekennzeichnet, daß** das Verriegelungsorgan (43) und der Steuermechanismus (24) dem zweiten Element zugeordnet sind, und das Verriegelungsorgan dazu vorgesehen ist, mit einer Abstützfläche (14b) zusammenzuwirken, die sich radial von der Außenfläche des Ansatzes des ersten Elementes zur Außenseite dieses Ansatzes hin erstreckt.

8. Verbindungseinrichtung nach Anspruch 7, **dadurch gekennzeichnet, daß** die Abstützfläche, die dazu vorgesehen ist, mit dem Verriegelungsorgan zusammenzuwirken, von der der Sockelplatte des ersten Elementes gegenüberliegenden Seitenfläche (14b) einer Rippe (14) mit abgeschrägten Seitenflächen (14a, 14b) gebildet wird, welche auf der Außenfläche des Ansatzes des ersten Elementes vorsteht.

9. Verbindungseinrichtung nach Anspruch 7, **dadurch gekennzeichnet, daß** sich die auf der Außenfläche des Ansatzes des ersten Elementes vorstehende Rippe mit abgeschrägten Seitenflächen über den gesamten Umfang dieses Ansatzes erstreckt.

10. Verbindungseinrichtung nach Anspruch 8 oder Anspruch 9, **dadurch gekennzeichnet, daß** das Verriegelungsorgan einstückig mit dem Steuermechanismus ist und aus mindestens einer Rippe (43) mit abgeschrägten Seitenflächen (43a, 43b) besteht, wobei eine dieser Seitenflächen (43b) derjenigen Seitenfläche der Rippe des Ansatzes des ersten Elementes zugeordnet ist, die als Abstützfläche dienen soll und auf der dem Ansatz des zweiten Elementes gegenüberliegenden Fläche des Steuermechanismus vorsteht.

11. Verbindungseinrichtung nach einem der Ansprüche 6 bis 10, **dadurch gekennzeichnet, daß** der Steuermechanismus einen Arm (24) aufweist, der verschwenkbar um eine Achse gelagert ist, die von einem Zapfen (25) gebildet wird, den dieser Arm aufweist und der in eine in der Sockelplatte des zweiten Elementes vorgesehene Öffnung (26) eingreift.

12. Verbindungseinrichtung nach Anspruch 11, **dadurch gekennzeichnet, daß** das zweite Element Mittel zum Halten und Führen des Armes in einer zur Ebene seiner Sockelplatte parallelen Ebene aufweist.

13. Verbindungseinrichtung nach Anspruch 12, **dadurch gekennzeichnet, daß** die Mittel zum Halten und Führen des Armes mindestens eine Lasche (27, 28, 29) aufweisen, die auf der der Sockelplatte des zweiten Elementes gegenüberliegenden Fläche des Armes vorsteht und ein freies Ende mit einem zurückgebogenen Abschnitt aufweist, welcher eine Abstützfläche (27a, 28a, 29a) darstellt, und dazu ausgelegt ist, ein in der Sockelplatte des zweiten Elementes vorgesehenes Langloch (31, 32, 33) zu durchsetzen, so daß die Abstützfläche auf einer Fläche (31a, 32a, 33a) der Sockelplatte gleitet, die auf der dem Arm entgegengesetzten Seite liegt.

14. Verbindungseinrichtung nach einem der Ansprüche 11 bis 13, **dadurch gekennzeichnet, daß** der Arm auf seiner der Sockelplatte des zweiten Elementes gegenüberliegenden Seite mindestens ein vorspringendes Teil (40) aufweist, das dazu vorgesehen ist, mit einer auf dem Außenrand dieser gleichen Sockelplatte vorgesehenen Nase (41) zusammenzuwirken, um das Blockieren des Verriegelungsorgans im Verriegelungszustand zu bewirken.

15. Verbindungseinrichtung nach einem der Ansprüche 1 bis 14, **dadurch gekennzeichnet, daß** das zweite Element der Verbindungseinrichtung des weiteren Mittel (48) aufweist, die dazu geeignet sind, die radiale Verschiebung der Basis der ringförmigen Lippe in Richtung auf den Ansatz des ersten Elementes zu begrenzen, wenn die beiden Elemente der Verbindungseinrichtung zusammengesetzt sind.

16. Verbindungseinrichtung nach einem der Ansprüche 1 bis 5, **dadurch gekennzeichnet, daß** das Verriegelungsorgan (53) und der Steuermechanismus (54) dem ersten Element der Verbindungseinrichtung zugeordnet sind, und daß das Verriegelungsorgan dazu vorgesehen ist, mit einer Abstützfläche (51a) zusammenzuwirken, die sich radial von der Außenfläche des Ansatzes des zweiten Elementes in Richtung auf die Innenseite dieses Ansatzes hin erstreckt.

17. Verbindungseinrichtung nach Anspruch 16, **dadurch gekennzeichnet, daß** die zum Zusammenwirken mit dem Verriegelungsorgan bestimmte Abstützfläche von derjenigen Wand (51a) einer in der Dicke des Ansatzes des zweiten Elementes vorgesehenen Aussparung (51) gebildet wird, die der Sockelplatte des zweiten Elementes gegenüberliegt,.

18. Verbindungseinrichtung nach Anspruch 17, **dadurch gekennzeichnet, daß** sich die in der Dicke des Ansatzes des zweiten Elementes vorgesehene Aussparung über den gesamten Umfang dieses Ansatzes erstreckt.

19. Verbindungseinrichtung nach einem der Ansprüche 16 bis 18, **dadurch gekennzeichnet, daß** die Sockelplatte des zweiten Elementes zwei Stifte aufweist, die dazu geeignet sind, die Drehung eines Elementes bezogen auf das weitere zu begrenzen, wenn die beiden Elemente der Verbindungseinrichtung zusammengesetzt sind.

20. Verbindungseinrichtung nach einem der Ansprüche 17 bis 19, **dadurch gekennzeichnet, daß** das Verriegelungsorgan einstückig mit dem Betätigungsmechanismus ist und aus einer Stange (53) besteht, die in einer zylindrischen Öffnung (52) angeordnet ist, welche den Ansatz des ersten Elementes durchsetzt, und von der ein Ende dazu geeignet ist, in die in der Dicke des Ansatzes des zweiten Elementes vorgesehene Aussparung eingesetzt zu werden, während ihr weiteres Ende fest mit dem Betätigungsmechanismus verbunden ist.

21. Verbindungseinrichtung nach Anspruch 20, **dadurch gekennzeichnet, daß** die Stange über ihre gesamte Länge oder einen Teil ihrer Länge mit einem Gewinde versehen ist, und der Betätigungsmechanismus von einer Schraubhülse (54) oder dergleichen gebildet wird, die dazu geeignet ist, ihre Verschiebung mittels Drehung in der zylindrischen Öffnung zu ermöglichen, in der sie angeordnet ist.

22. Stomavorrichtung mit einem Beutelhalter zum Befestigen um eine künstliche Öffnung des Körpers eines Anwenders, sowie einem Beutel zum Auffangen von Körpersubstanzen, welcher abnehmbar an dem Beutelhalter angebracht werden kann, **dadurch gekennzeichnet, daß** sie eine Verbindungseinrichtung nach einem der Ansprüche 1 bis 21 aufweist.

## Claims

1. Connector for stoma apparatus comprising two elements (10, 20) able to be assembled in a detachable manner and each having a seat (11, 21) provided with a central opening and a tubular joining piece (12, 22) surrounding the said opening, and wherein the joining piece (12) of the first element (10) has, on its inner face, an elastically deformable annular lip (18), the free end (19) of which is directed towards the plane of the seat (11) of the said element, whereas the joining piece (22) of the second element (20) has, on its outer face, means (44) for axial retention of the free end of this lip, the said connector being **characterised in that** it has a locking member (43, 53) and **in that** the means for axial retention of the free end of the annular lip occupy a sector of the circumference of the joining piece of the second element, of which the middle point (45) is located diametrically opposite the said locking member when the two elements of this connector are under assembly and disassembly conditions.

2. Connector as claimed in claim 1, **characterised in that** the means for axial retention of the free end of the annular lip occupy a sector of the circumference of the joining piece of the second element corresponding to an angle of between about 180° to 320°.

3. Connector as claimed in claim 1 or claim 2, **characterised in that** the means for axial retention of the free end of the annular lip are formed by an edge (44) extending radially from the outer face of the joining piece of the second element towards the outside of this joining piece.

4. Connector as claimed in claim 3, **characterised in that** the edge is a diminishing edge, the width of which is at a maximum at the middle point (45) of the sector which it occupies, and diminishes progressively towards the ends (46, 47) of this sector.

5. Connector as claimed in any one of claims 1 to 4, **characterised in that** it has means for keeping the locking member in the locking condition.

6. Connector as claimed in any one of claims 1 to 5, **characterised in that** since the locking member (43, 53) is associated with one of the two elements of the connector, it is provided to cooperate, in response to the actuation of a control mechanism (24, 54) associated with the same element as it is associated with, with an abutment surface provided on the joining piece of the other of the two elements of the connector.

7. Connector as claimed in claim 6, **characterised in that** the locking member (43) and the control mechanism (24) are associated with the second element and **in that** the locking member is provided to cooperate with an abutment surface (14b) extending radially from the outer face of the joining piece of the first element towards the outside of this joining piece.

8. Connector as claimed in claim 7, **characterised in that** the abutment surface intended to cooperate with the locking member is formed by the cant (14b) located facing the seat of the first element, of a rib (14) with cants (14a, 14b) protruding beyond the outer face of the joining piece of this first element.

9. Connector as claimed in claim 7, **characterised in that** the rib with cants protruding beyond the outer face of the joining piece of the first element extends over the whole circumference of this joining piece.

10. Connector as claimed in claim 8 or claim 9, **characterised in that** the locking member forms one piece with the control mechanism and is formed by at least one rib (43) with cants (43a, 43b), one of which cants (43b) is paired with the cant of the rib of the joining piece of the first element intended to serve as an abutment surface, and which protrudes beyond the face of the said control mechanism located facing the joining piece of the second element.

11. Connector as claimed in any one of claims 6 to 10, **characterised in that** the control mechanism has an arm (24) mounted to as to be able to pivot about an axis formed by a journal (25) which it has and which is engaged in an orifice (26) provided in the seat of the second element.

12. Connector as claimed in claim 11, **characterised in that** the second element has means for holding and guiding this arm in a plane parallel to that of its seat.

13. Connector as claimed in claim 12, **characterised in that** the means for holding and guiding the arm include at least one lug (27, 28, 29) protruding beyond the face of this arm located facing the seat of the second element, having a free end with a return defining an abutment face (27a, 28a, 29a) and adapted to pass through a button hole (31, 32, 33) provided in the seat of the second element so that the said abutment face slides on a face (31a, 32a, 33a) of the said seat located on the opposite side to the said arm.

14. Connector as claimed in any one of claims 11 to 13, **characterised in that** the said arm has, on its face located facing the seat of the second element, at least one protruding piece (40) adapted to cooperate with a tip (41) provided on the outer edge of this same seat to ensure that the locking member is kept in the locking position.

15. Connector as claimed in any one of claims 1 to 14, **characterised in that** the second element of the connector additionally has means (48) able to limit the radial displacement of the bottom of the annular lip in the direction of the joining piece of the first element when the two elements of this connector are assembled.

16. Connector as claimed in any one of claims 1 to 5, **characterised in that** the locking member (53) and the control mechanism (54) are associated with the first element of this connector and **in that** when the locking member is provided to cooperate with an abutment surface (51 a) extending radially from the outer face of the joining piece of the second element towards the inside of this joining piece.

17. Connector as claimed in claim 16, **characterised in that** the abutment surface intended to cooperate with the locking member is formed by the wall (51a) located facing the seat of the second element of a recess (51) provided in the thickness of the joining piece of this second element.

18. Connector as claimed in claim 17, **characterised in that** the recess provided in the thickness of the joining piece of the second element extends over the whole circumference of this joining piece.

19. Connector as claimed in any one of claims 16 to 18, **characterised in that** the seat of the second element has two studs able to limit the rotation of one element with respect to the other when the two elements of this connector are assembled.

20. Connector as claimed in any one of claims 17 to 19, **characterised in that** the locking member forms one piece with the actuating mechanism and is formed by a rod (53) which is placed in a cylindrical opening (52) passing through the joining piece of the first element, one end of which is able to be housed in the recess provided in the thickness of the joining piece of the second element while its other end is fixedly attached to the said actuating mechanism.

21. Connector as claimed in claim 20, **characterised in that** the rod is threaded over all or part of its length and the actuating mechanism is formed by a roller (54) or the like, able to permit its displacement by rotation in the cylindrical opening in which it is placed.

22. Stoma apparatus comprising a pocket support intended to be fixed around an artificial opening in the body of a user, as well as a pocket for collecting bodily materials able to be removably collected in the said pocket support, **characterised in that** it comprises a connector as claimed in any one of claims 1 to 21.
